# EUROPEAN PATENT APPLICATION

(11) **EP 2 243 422 A1**
(43) Date of publication of application: **27.10.2010**
(21) Application number: 09158661.0
(22) Date of filing: 23.04.2009
(51) Int. Cl.: A61B 5/00, A61B 5/11, G01K 1/02

(54) **Method, system and sensor for monitoring an infant lying in a bed**

(71) Applicant: IMDS R&D BV, 9751 VD Haren (NL)
(72) Inventor: Schulting, Edwin Alexander, 9751 VD Haren (NL)
(74) Representative: Hatzmann, Martin

(57) **Abstract**

For monitoring an infant (1; 51) lying in a bed, continuously or periodically a temperature is measured in a confined layer of air (67, 68) that is shielded from the infant (1; 51) by at least one first thermally insulating layer (8; 65) and shielded from the environment by at least one second thermally insulating layer (3; 53, 58). The second thermally insulating layer (3; 53, 58) insulates a portion of the infant (1; 51) and the first thermally insulating layer (8; 65) from the environment. A warning signal is generated if the measured temperature exceeds a threshold temperature. The threshold temperature is lower than 36 °C which is significantly lower than a body temperature of an infant that would make a warning signal desirable. A system and a sensor for use in the method according to the invention are also described.

## Description

### FIELD AND BACKGROUND OF THE INVENTION

The invention relates to a method, a system and to a sensor for monitoring an infant lying in a bed.

The sudden infant death syndrome (SIDS) is an important cause of death among infants one month to one year old. For instance in the United States SIDS is estimated to be responsible for roughly one death per 2,000 births (2,247 in 2004 - Thomas Hargrove and Lee Bowman, Scripps Howard News Service, October 8, 2007).

One factor affecting the risk of SIDS seems to be the sleep position of the infant. Infants that sleep on their backs (supine position) instead of their stomachs (prone position) tend to have lower arousal thresholds and less Slow-Wave Sleep (SWS) compared to infants who sleep on their stomachs, so the risk of the infant being unable to arouse, for instance when they have an incidence of sleep apnea, is decreased. Also, small infants with little or no control of their heads may, while face down, inhale their exhaled breath (high in carbon dioxide) or smother themselves on their bedding.

It is therefore generally recommended that healthy infants be positioned to sleep on their back or side (lateral position), instead of their stomach.

Also body temperature seems to affect the risk of SIDS. In particular in infants of up to six months body temperature control in the event of insufficient heat dissipation is still in development, so that it is important to ensure that the room temperature is not too high, exposure to radiation such as solar radiation and radiation from room heating elements is limited and that the extent to which the infant is thermally insulated from the surroundings is adequate in view of the circumstances.

Systems for monitoring the position of a sleeping infant are disclosed in International patent application WO 2007/074345, U.S. patent 6 468 234 and U.S. patent application 2007/0260209. Of these systems, the systems disclosed in International patent application WO 2007/074345 in U.S. patent 6 468 234 include sensors for detecting the body temperature of the infant. The system disclosed in International patent application WO 2007/074345 also includes sensors for detecting the temperature of the environment. Other systems for monitoring the body temperature of a sleeping infant are disclosed in U.S. patent 6 686 843 and U.S. patent application 2002/0013538.

### SUMMARY OF THE INVENTION

It is an object of the present invention to provide a simple solution for monitoring a sleeping infant, which generates a warning signal in the event of insufficient heat dissipation from the infant as well as in the event of a too high body temperature of the infant.

According to the invention, this object is achieved by providing a method according to claim 1. The invention can also be embodied in a system according to claim 8.

By continuously or periodically measuring a temperature in a confined layer of air that is shielded from the infant by at least one first heat insulating layer and shielded from the environment by at least one second heat insulating layer, the second heat insulating layer insulating a portion of the infant and the first heat insulating layer from the environment, the temperature is measured in a location which is affected by the infants body temperature as well as by the heat dissipation to the environment. At a given body temperature, the temperature in a layer of air near the infants body mainly determines how much heat is transported away from the infants body. However, the temperature in the layer of air near the infant's body is also influenced by the temperature of the infant's body. In view of the required heat transport away from the infant's body, the temperature in the layer of air near the infant's body should be well below the infants desired body temperature. Accordingly, by generating a warning signal in response to the measured temperature in a layer of air near the infant's body exceeding a threshold temperature lower than 36 °C, a single measurement provides both an indication for insufficient heat dissipation and for a too high body temperature. It is noted that the threshold temperature lower than 36 °C is significantly lower than even the skin temperature of the infant at which a warning for overheating would be desirable.

According to a further aspect of the invention, a simple and compact sensor for monitoring both the sleeping position of the infant and the temperature in a layer of air near the infant's body is obtained by providing a sensor according to claim 12.

Particular elaborations and embodiments of the invention are set forth in the dependent claims.

Further features, effects and details of the invention appear from the detailed description and the drawings.

### BRIEF DESCRIPTION OF THE DRAWINGS

Fig. 1 is a schematic representation of a sleeping baby and a first example of a system according to the invention;
Fig. 2 is a schematic representation of an abdominal portion of a body of an infant and a second example of a system according to the invention;
Fig. 3 is a graph showing temperatures of an infant, of layers of air near the infant and of the room around the infant; and
Fig. 4 is a schematic representation of a first example of a sensor for sensing temperature of a layer of air near an infant and the position of the infant in an inverted position;
Fig. 5 is a schematic representation of the sensor of Fig. 4 in its normal position;
Fig. 6 is a schematic representation of a second example of a sensor for sensing temperature of a layer of air near an infant and the position of the infant in an inverted position;
Fig. 7 is a schematic representation of the sensor of Fig. 6 in its normal position;
Fig. 8 is a schematic representation of a third example of a sensor for sensing temperature of a layer of air near an infant and the position of the infant in an inverted position; and
Fig. 9 is a schematic representation of the sensor of Fig. 8 in its normal position.

### DETAILED DESCRIPTION

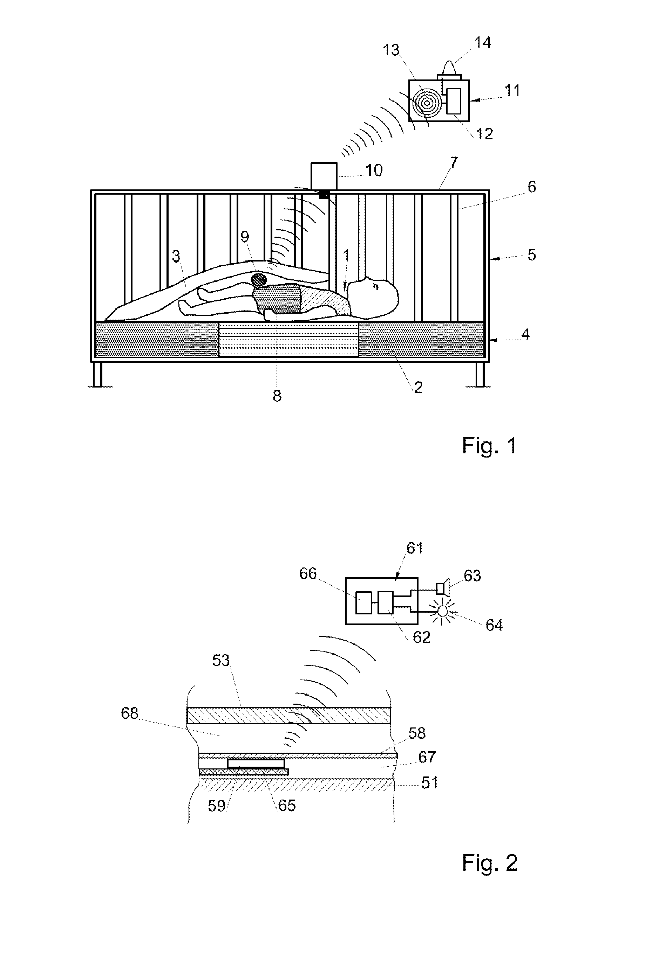

In Fig. 1, an infant 1 asleep on a mattress 2 under a duvet 3 is shown. The mattress 2 and the infant 1 are located on a cot 4 having a safety barrier 5 around the mattress 2 in the form of uprights 6 connected by rails 7. The infant is wearing a romper (playsuit) 8 without legs and with short sleeves and, underneath, a diaper (not shown).

For monitoring the infant 1 lying in the bed 2, a system is provided which, according to the present example, is divided in three parts.

Firstly, a temperature sensor 9 for periodically generating a signal representing a temperature of the sensor 9. The sensor has an operating temperature range at least including a temperature range of 27-32 °C. According to the present example, the sensor 9 is capable of generating first, wireless signals. Secondly, a relay station 10 for receiving the first signals from the sensor 10 and transmitting corresponding second signals. Thirdly, the system includes a receiver station 11 for receiving signals from the relay station 10. The relay station 10 is located close to the sensor 9, so that relatively weak signals that can only be received within a relatively small range from the sensor 9 can nevertheless be received reliably. That the signals from the sensors 9 are received within a small range provides the advantage, that a low power transmitter in the sensor 9 is sufficient for outputting signals that are reliably received and that only low power radio signals need to be generated in the vicinity of newborn and very young infants.

The temperature sensor 9 is to some extent insulated from the body of the infant 1 by first heat insulating layers constituted by the romper 8 and the diapers (not shown) and to some extent insulated from the environment by a second heat insulating layer in the form of the duvet 3. Instead of a duvet, the second heat insulating layer may also be another type of bedding, such as a blanket or, if the ambient room temperature is relatively high, a sheet.

The receiver station 11 is equipped with a signal processor 12 remote from the sensor 9 and the relay station 10. The receiver station may for instance be positioned in another room or floor than the infant, for instance in a room where the infant's parents are. When in operative condition the signal processor 12 receives signals from the sensor 9 via the relay station 10. The signal processor 12 is arranged for outputting a command signal in response to the measured temperature exceeding a threshold temperature. The threshold temperature is lower than 36 °C, but preferably higher than 29 °C. Depending on the location of the temperature sensor, a different threshold temperature may be preferable. The more the sensor is isolated from the infant 1, the lower the threshold temperature should be. Generally, an upper threshold temperature below 35 °C and more preferably below 34 °C and above 30 °C and more preferably above 32 °C is advantageous for combining a good sensitivity to both a too high infant body temperature and to a too low heat transfer rate from the infant to the environment at room temperatures in a usual range of 15 to 22 °C.

Since normal body temperatures of sleeping children vary between individuals and over time (typically the temperature decreases gradually and slightly during sleeping), it is preferred that each threshold value is at least to some extent adapted to the average temperature measured over a duration of time (for instance three to 12 hours) in which no alarm was generated. For instance, the reference temperature may initially (when the device is used for the first time or after a reset) be a preset reference temperature and the temperature range which does not cause the generation of an alarm signal may also be preset. During use, a temperature curve of the infant may be stored. Preferably, the curve represents a moving average or an aggregation of moving averages and the width of allowable deviations from the expected temperature curve may be adapted to the typical bandwidth of deviations of the particular child that do not indicate a situation requiring attention. After a child has been put to bed, the bed is initially relatively cold so a quick rise of the temperature will be found and the child may still be awake, so temperature variations may also be caused by movements of the child. Therefore, preferably no alarm is generated during the first 15 minutes after the system has been activated.

Generically applicable rules for the threshold values may prescribe that the adaptively determined threshold temperature is within a certain range, for instance between 31 and 35 °C for the upper threshold value and between 24 and 28 °C for the lower threshold value.

The receiver station 11 is further equipped with signaling devices in the form of a buzzer 13 and an alarm light 14 communicating with the a signal processor 12 and arranged for generating a warning signal in response to a command signal received from the signal processor 12.

In operation, the infant 1 is monitored by periodically, for instance every 10 or 20 seconds, measuring a temperature in a confined layer of air by the temperature sensor 9, which is shielded from the infant 1 by at least one first heat insulating layer, in this example constituted by the romper 8 and the diaper, and shielded from the environment by at least one second heat insulating layer, in this example constituted by the duvet 3. The duvet 3 insulating a portion of the infant other than its head and upper torso portion and also a portion of the romper 8 from the environment.

A warning signal is generated by the buzzer 13 and the signaling light 14 in response to the measured temperature exceeding the threshold temperature, which has been set to be lower than 36 °C and preferably within ranges as discussed above.

In Fig. 2, an alternative example of a system according to the invention is shown with a sensor 59 in a different position than the sensor 9 in Fig. 1. An abdominal portion 59 of an infant is partially covered by a waist band 65 of a diaper. Over the diapers, the infant wears a romper 58. A duvet 53 covers the infant from the shoulders down. The duvet 53 may be filled with down, silk, wool, cotton, or artificial fibers (such as polyester batting or other artificial material) or other heat insulating material which allows some dissipation of humidity to the environment through the material.

A receiver station 61 is provided with a receiver 66 for receiving signals from the temperature sensor 59 and with a signal processor 62 for processing the signals received from the temperature sensor 59. The signal processor 62 is arranged for generating and outputting command signals to a sound generator 63 and to a warning light 64, but may also be arranged to output signals to the sound generator 63 or to the warning light 64 only. For instance, only the warning light may be activated to indicate that the child is bedded relatively (but not alarmingly) warmly or freshly. In the present example, the receiver station 61 receives signals from the temperature sensor 59 directly. For warning persons remote in another room than the infant, it may be provided that the sound generator generates a relatively loud signal in response to a command from the signal processor. Generating such a loud signal may also be advantageous for immediately causing the infant to wake up if a warning condition occurs, even before the parent or other child-minder has arrived. To avoid unnecessary stressful situations, the loud sound generated by the loudspeaker may be a pleasant sound, such as music, a friendly voice or certain sounds from nature.

In the present example, the temperature sensor 59 is arranged to the outside of the diapers 65 and inside the romper 58. Thus, of the first and second heat insulating layers between which the temperature sensor is arranged, a portion of the diapers 65 constitutes the first heat insulating layer and the second heat insulating layer is part of a garment in the form of the romper 58. Because an upper end of the diaper 65, which thermally shields the temperature sensor from direct contact with the skin of the infant 51, ends in the layer of air 67 between the skin of the infant 51 and the romper 58, a representative measurement of the temperature of the layer of air 67 between the skin of the infant 51 and the romper 58 can be obtained. If it is desired that the measurement result is influenced more by ambient temperature and the insulation provided by the outer layers 53, 58, the temperature sensor can be placed between the romper 58 and the duvet 53 (as in the example shown in Fig. 1) or the temperature sensor can be arranged to include a thermal insulation in a side of the temperature sensitive element facing the infant, so that the measured temperature is more strongly affected by the temperature on the outside of the sensor than by the temperature on the inside of the sensor.

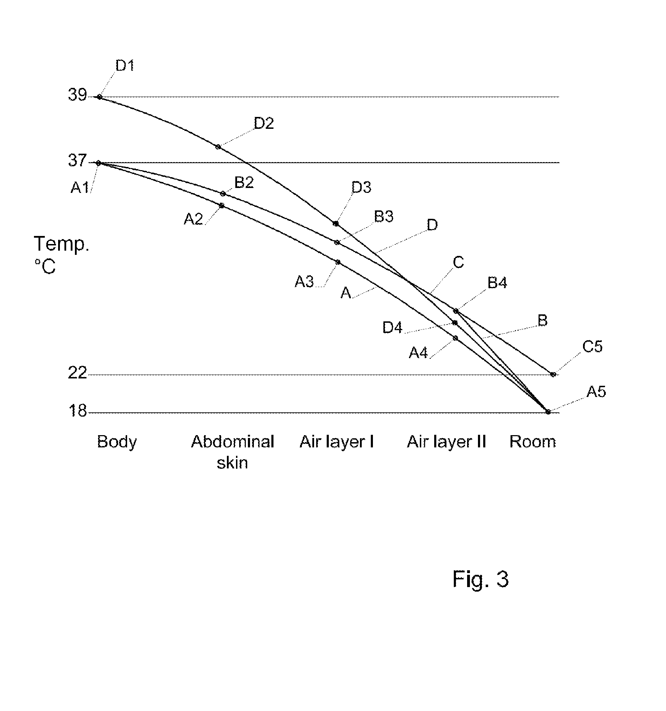

Fig. 3 illustrates an example of how monitoring both body temperature changes and changes in the heat transfer rate to the environment by measuring only one temperature in a layer of air near the infant that is to some extent thermally insulated from the infant and to some extent thermally insulated from the environment.

The graph A represents an example of a normal temperature gradient in which:
A₁ = a body temperature
A₂ = an abdominal skin temperature,
A₃ = a temperature of an air layer I between a diaper and a romper,
A₄ = a temperature of an air layer II between a romper and a blanket, and
A₅ = an ambient room temperature.

The graph B (A₁, B₂, B₃, B₄, A₅) represents a graph of a situation in which the blanket provides too much thermal insulation. This causes the temperature difference between B₄ (the temperature of the air layer II between the romper and the blanket) and A₅ (the ambient room temperature) to become larger. Since the room temperature remains the same, the resulting temperatures B₃ and B₄ of the first and second air layers, which are higher than the corresponding temperatures A₃ and A₄ of the first and second air layers in a normal temperature gradient, each constitute an indication of insufficient heat transfer to the environment.

The graph C (A₁, B₂, B₃, B₄, C₅) represents a graph of a situation in which the elevated temperature B₄ of the air layer II between the romper and the blanket is not caused by insufficient heat transfer via the blanket, but because of a too high ambient room temperature C₅. Thus, the resulting temperatures B₃ any B₄ of the first and second air layers, which are higher than the corresponding temperatures A₃ and A₄ of the first and second air layers in a normal temperature gradient, do also provide an indication to generate a warning in the event of insufficient heat transfer to the environment because of a too high ambient room temperature.

The graph D (D₁, D₂, D₃, D₄, A₅) represents a graph of a situation in which the infant is suffering from a fever, but the ambient room temperature is A₅ normal. In this situation, the increased temperature difference between the body temperature D₁ and the ambient room temperature A₅ is evened out over the temperatures in the intermediate positions D₂, D₃ and D₄. This results in temperatures D₃ and D₄ higher than the corresponding temperatures A₃ and A₄ of a normal temperature gradient. Thus the increased temperatures D₃ of and D₄ of the first and second air layers, which are higher than the corresponding temperatures A₃ and A₄ of the first and second air layers in a normal temperature gradient, each also constitute an indication of fever.

Thus, measuring the temperature at a single location in any of the air layers between garments or between a garment and a blanket or other cover, near the body of an infant provides sufficient indication for obtaining a warning in the event of any of the following temperature conditions to which an increased risk of SIDS is associated:
- fever,
- too much thermal insulation,
- too much heat from a heat source near the infant, such as a hot water bottle or an electric blanket, and
- too high ambient room temperature.

Too much thermal insulation may be caused by the bed cover, the mattress and or the clothing.

A too high temperature at the outside of the blanket or other cover may also or partially be caused by solar and/or heat radiation. However, for further improving the reliability and the specificity of the monitoring, other sensors may also be added. For instance, the ambient temperature can quite easily be measured using a temperature sensor in the relay station 10.

The signal processor is preferably further arranged for outputting a command signal in response to a temperature rise or fall exceeding a threshold rate of change per unit of time of 0.06 °C / min. Generating a temperature in response to changes of temperature exceeding a given rate of change per unit of time is particularly valuable for generating a warning in situations indicating an increased risk of heat shock and heart problems. Heat shocks are typically developed with a rapid increase of body heat emission. When the baby develops a heat shock the environment around the infant will show a rapid increase in this temperature in response to which the signal processor causes an alarm signal to be generated. In the event of a heart beat slow down or stop, the temperature of the infant will decrease resulting in a rapidly reduced temperature. The rate of change in response to which an alarm signal is caused may be different for increases than for decreases of temperature. To avoid false alarms, it is preferably required that the rate of change is determined over a window of time or required to be sustained over a period of time of at least 20 minutes. The duration of the period of time over which the rate of change is determined or required may be reduced when the measured temperature is more remote from a normal mean value.

The risk of SIDS may further be reduced by additionally providing the system with a position sensor for continuously or periodically monitoring the position of the trunk of the infant 1 and generating a warning signal in response to a change of the position exceeding a rotation about its longitudinal axis of at least 135 degrees.

According to the examples discussed below, the temperature sensor is integrated with the position sensor in a single sensor, so that only a single sensor needs to be applied to the infant's body. However, in the method and system according to the invention the position sensor may also be provided as a separate device to be applied to the body of the infant or in the vicinity of the infant, for instance in the form of pressure sensors to be arranged under the infant or a camera to be arranged above and/or to the side of the infant.

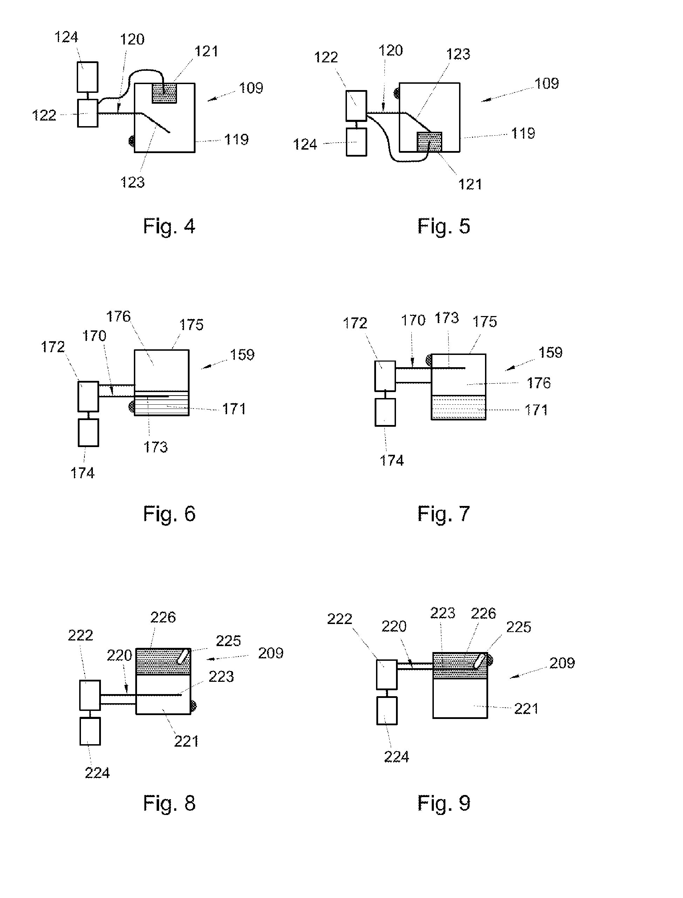

In Figs. 4 and 5, an example of a sensor 109 for detecting both a temperature in a layer of air near the infant and changes of the position of the infant is shown. The sensor 109 includes a frame 119, a sensing element 120 having a temperature dependent conductivity, a conductive contact 121, a circuitry 122 for generating a signal depending on the resistance encountered by a current through the sensing element 120, which circuitry 122 is connected to the sensing element 120 and to the contact 121. The circuitry is also connected to a transmitter 124 for outputting a signal representing the measured temperature to the transmitter 124, in response to which signal the transmitter 124 transmits a radio signal representing that measured temperature, which signal is to be received by a receiver station.

The sensing element 120 has an end portion 123 that is movable relative to the conductive contact 121, which is fixed to the frame 119, for displacement in response to changes of orientation of the sensor 109, between a position in mutual electrically conductive contact and a position in mutual electrical isolation.

If the sensor is in the position shown in Fig. 5, the movable portion 123 of the sensing element 120 is in contact with the conductive contact 121 and the resistance measured over the sensing element 120 and the conductive contact 121 is a measure for the temperature of the sensor 109. In response, the circuitry 122 generates and outputs a signal that indicates that the sensing element 120 is in contact with the conductive contact 121, which signal also represents a sensed temperature. Accordingly, the position shown in Fig. 5 is an upright position in which the sensor 109 should approximately be when the infant is lying in the supine position. If the sensor 109 is in the upside-down position shown in Fig. 4, the movable portion 123 of the sensing element 120 is free from the conductive contact 121 so the circuit including the sensing element 120 and the conductive contact 121 is interrupted. This condition causes the circuitry 122 to output a signal to the transmitter 124 indicating that the sensing element 120 is not in contact with the conductive contact 121, i.e. that the sensor 109 is in an upside-down position. This causes the transmitter 124 to transmit a radio signal also representing the upside-down condition of the sensor 109, in response to which signal a warning signal is generated by the receiver station.

The sensor 159 shown in Figs. 6 en 7 has a housing 175 enclosing a chamber 176. The conductive contact includes a volume 171 of mutually loose, electrically conductive particles enclosed in the chamber 176, occupying a portion of the chamber 176 and leaving the remainder of the chamber 176 free. The sensing element 170 has a contact surface 173 positioned in the chamber 176 in such a position that the volume of conductive particles is displaceable relative to the contact surface 173 in response to changes of orientation of the sensor 159, between a position in electrically conductive contact with the contact surface 173 (Fig. 6) and an upside-down position in electrical isolation from the contact surface (Fig. 7). In the position shown in Fig. 6, current through the circuit constitutes an indicator of the temperature of the sensor 159 and that the sensor is in an upright position. In the position shown in Fig. 7, the circuit including the sensing element 170 and the contact material 121 is interrupted, which indicates that the sensor is upside-down or at least tilted to a large degree. This indicates that the infant is not in the supine position, so it would be advisable to check the condition of the infant. The circuitry 172 and the transmitter 174 are arranged so that the transmitter 174 generates a signal to warn the parents if the sensor 159 is in the position shown in Fig. 7.

The sensor 209 shown in Figs. 8 en 9 also has a housing 225 enclosing a chamber 226. The conductive contact includes a volume 221 of a conductive polar liquid, for instance as a solution of a salt in water, in the housing 226, occupying a portion of the chamber 226. The remainder of the chamber is filled with a non-conductive liquid that does not mix with the conductive liquid and has a lower specific mass, for instance an oil or other apolar liquid. The sensing element 220 has a contact surface 223 positioned in the chamber 226 in such a position that the volume of the conductive liquid is displaceable relative to the contact surface 223 in response to changes of orientation of the sensor 209, between a position in electrically conductive contact with the contact surface 223 (Fig. 8) and an upside-down position in electrical isolation from the contact surface (Fig. 9). In the position shown in Fig. 8, current through the circuit constitutes an indicator of the temperature of the sensor 209 and that the sensor is in an upright position. In the position shown in Fig. 7, the circuit including the sensing element 220 and the contact material 121 is interrupted, which indicates that the sensor is upside-down or at least tilted to a large degree. This indicates that the infant is not in the supine position, so it would be advisable to check the condition of the infant. The circuitry 222 and the transmitter 224 are arranged so that the transmitter 224 generates a signal to warn the parents if the sensor 209 is in the position shown in Fig. 7.

The skilled person will readily appreciate, that within the framework of the invention as set forth in the claims, many other examples than the examples discussed above are conceivable. For instance, the temperature and/or the position may be monitored continuously instead of periodically and the sensor may be connected with the signal processor via a wire. The relay station and/or the receiving station may be arranged for communication with a local or wide area network either via a communication device or directly. The network may be a wired or wireless network and be a computer network such as a WiFi or Ethernet network or a telephone network such as a PSTN, GSM or DECT network.

## Claims

1. A method for monitoring an infant (1; 51) lying in a bed, the method comprising:
continuously or periodically measuring a temperature in a confined layer of air (67, 68) that is shielded from the infant (1; 51) by at least one first thermally insulating layer (8; 65) and shielded from the environment by at least one second thermally insulating layer (3; 53, 58), said second thermally insulating layer (3; 53, 58) insulating a portion of the infant (1; 51) and the first thermally insulating layer (8; 65) from the environment, and
generating a warning signal in response to the measured temperature exceeding a threshold temperature, the threshold temperature being lower than 36 °C.

2. A method according to claim 1, wherein the first thermally insulating layer (65) is part of an absorbent article such as a diaper.

3. A method according to claim 1 or 2, wherein the second thermally insulating layer (58) is part of a garment.

4. A method according to any one of the preceding claims, wherein the second thermally insulating layer (3; 53) is part of a bedding, such as a blanket, a duvet or a sheet.

5. A method according to any one of the preceding claims, wherein a warning signal is also generated in response to a temperature rise or fall exceeding a threshold rate of change per unit of time of 0.06 °C / min.

6. A method according to any one of the preceding claims, further comprising continuously or periodically monitoring the position of the trunk of the infant (1; 51) an generating a warning signal in response to a change of the position exceeding a rotation about its longitudinal axis of at least 135 degrees.

7. A method according to any one of the preceding claims, wherein the temperature is measured in a single location only.

8. A system for monitoring an infant (1; 51) lying in a bed, the system comprising:
a temperature sensor (9; 59; 109; 159; 209) for continuously or periodically generating a signal representing a temperature of or detected by the sensor (9; 59; 109; 159; 209),
a signal processor (12; 62) remote from and receiving signals from the temperature sensor (9; 59; 109; 159; 209) when in operative condition, the signal processor (12; 62) being arranged for outputting a command signal in response to the measured temperature exceeding a threshold temperature, the threshold temperature being lower than 36 °C, and
a signaling device (13, 14; 63, 64) communicating with the signal processor (12; 62) when in operative condition and arranged for generating a warning signal in response to a command signal received from the signal processor (12; 62).

9. A system according to claim 8, wherein the signal processor (12; 62) is further arranged for outputting a command signal in response to a temperature rise or fall exceeding a threshold rate of change per unit of time of 0.06 °C / min.

10. A system according to claim 7 or 8, further comprising a position sensor (109; 159; 209) for continuously or periodically monitoring the position of the trunk of the infant (1; 51) and generating a warning signal in response to a change of the position exceeding a rotation about its longitudinal axis of at least 135 degrees.

11. A system according to claim 10, wherein the temperature sensor (109; 159; 209) is integrated with the position sensor (109; 159; 209).

12. An integrated sensor for sensing a temperature in a layer of air (67, 68) near an infant (1; 51) and a change of orientation of the infant (1; 51), comprising:
a sensing element (120; 170; 220) having a temperature dependent conductivity, and
a conductive contact (121; 171; 221),
wherein at least the conductive contact (171; 221) is displaceable relative to the sensing element (170; 220) or the sensing element (120) is movable relative to the conductive contact (121), for movement in response to changes of orientation of the sensor (9; 59; 109; 159; 209) between a position in mutual electrically conductive contact and a position in mutual electrical insulation, and
wherein the sensor includes circuitry for generating a signal indicating whether the sensing element (120; 170; 220) is in contact with the conductive contact (121; 171; 221) and representing a sensed temperature corresponding to the conductivity of the sensing element (120; 170; 220).

13. A sensor according to claim 12, further comprising a frame (119),
wherein the conductive contact (121) is mounted in a fixed position relative to the frame (119) and wherein the sensing element (120) is constituted by an elongate conductor structure (120), at least a portion (123) of the elongate conductor structure being movable between the position in electrically conductive contact with the contact (121) and a position in electrical insulation from the contact (121).

14. A sensor according to claim 12, further comprising a housing (175) enclosing a chamber (176), wherein the conductive contact includes a volume of mutually loose, electrically conductive particles (171) enclosed in the chamber (176), occupying a portion of the chamber (176) and leaving the remainder of the chamber (176) free, the sensing element (170) having a contact surface (173) positioned in the chamber (176) in such a position that the volume of conductive particles (171) is displaceable relative to the contact surface (173) in response to changes of orientation of the sensor (159), between a position in electrically conductive contact with the contact surface (173) and a position in electrical insulation from the contact surface (173).

15. A sensor according to claim 12, further comprising a housing (225) enclosing a chamber (226), wherein the conductive contact includes a volume of electrically conductive liquid (221) enclosed in the chamber (226), occupying a portion of the chamber (226) and leaving the remainder of the chamber (226) free, the sensing element (220) having a contact surface (223) positioned in the chamber (226) in such a position that the volume of conductive liquid (221) is displaceable relative to the contact surface (223) in response to changes of orientation of the sensor (209) between a position in electrically conductive contact with the contact surface (223) and a position in electrical insulation from the contact surface (223).
